# EUROPEAN PATENT APPLICATION

(11) **EP 1 818 018 A1**
(43) Date of publication of application: **15.08.2007**
(21) Application number: 07250538.1
(22) Date of filing: 09.02.2007
(51) Int. Cl.: A61B 10/02, A61B 18/02, A61B 17/32

(54) **Biopsy device mit cryogenic probe**

(30) Priority: 10.02.2006 US 351733
(71) Applicant: ETHICON ENDO-SURGERY, INC., Cincinnati, Ohio 45242 (US)
(72) Inventor: Voegele, James W., Cincinnati, OH 45249 (US)
(74) Representative: Tunstall, Christopher Stephen

(57) **Abstract**

A medical apparatus and method useful for obtaining a tissue sample are provided. The apparatus can include a rotating and translating cutter, a probe, and an outer sheath. The probe can be cooled, such as with a cryogenic substance. The sheath can shield surrounding tissue from the outer surface of the rotating cutter.

## Description

**Field of the Invention**

The present invention is related to biopsy devices, and more particularly, to a biopsy device employing a cryogenic substance.

**Background of the Invention**

The diagnosis and treatment of tissue is an ongoing area of investigation. Medical devices for obtaining tissue samples for subsequent sampling and/or testing are know in the art. For instance, a biopsy instrument now marketed under the tradename MAMMOTOME is commercially available from Ethicon Endo-Surgery, Inc. for use in obtaining breast biopsy samples.

The following patent documents disclose various biopsy devices and are incorporated herein by reference in their entirety: US 6,273,862 issued Aug 14, 2001; US 6,231,522 issued May 15, 2001; US 6,228,055 issued May 8, 2001; US 6,120,462 issued September 19, 2000; US 6,086,544 issued July 11, 2000; US 6,077,230 issued June 20, 2000; US 6,017,316 issued January 25, 2000; US 6,007,497 issued Dec. 28, 1999; US 5,980,469 issued Nov. 9, 1999; US 5,964,716 issued Oct 12, 1999; US 5,928,164 issued July 27, 1999; US 5,775,333 issued July 7, 1998; US 5,769,086 issued June 23, 1998; US 5,649,547 issued July 22, 1997; US 5,526,822 issued June 18, 1996, and US Patent Application 2003/0199753 published Oct 23, 2003 to Hibner et al.

A cryogenic probe is disclosed in US Patent No. 5,522,870, incorporated herein by reference.

Researchers in the medical device area continue to seek new and improved methods and devices for cutting, handling, and storing tissue samples.

**Summary of the Invention**

Applicant has recognized a need for an alternative method for taking a biopsy. In one embodiment, the present invention provides a device that incorporates a Joule-Thompson cryogenic probe with a core biopsy device, such as the Mammotome®, to take a tissue biopsy. The invention can be used to obtain a biopsy sample, such as a breast biopsy sample.

The invention, in one embodiment, may employ a cannula sheath and a cannular cutter. The cannula sheath can be non-rotating. The cannula sheath and the cannular cutter can be configured to be retracted so as to expose a cryogenic probe. The cryogenic probe can be disposed at least partially within the cannular cutter, and the probe can be movable axially with respect to the cannular cutter and the cannula sheath. In operation, a cryogenic gas or other cryogenic agent can be applied by the probe. The tissue can be adhered to the probe. The cryogenic agent can provide a change in the tissue's characteristics from a pliable state to a firm consistency. In one embodiment, the cannular cutter can be rotated as the cutter is advanced through the tissue after application of the cryogenic gas.

**Brief Description of the Figures**

Figure 1 is a top view of an embodiment of a device of the present invention showing the device handpiece.

Figure 2 is a top view of an embodiment of a device of the present invention showing the internal components of the device.

Figure 3 is a top view of an embodiment of a device of the present invention showing the cryogenic probe.

Figure 3A is a partial cross-sectional schematic of a distal portion of an embodiment of a cryogenic probe illustrating an internal tube for delivering cryogenic substance at the distal end of the probe.

Figure 4 is a top view of an embodiment of a device of the present invention showing the cannular cutter.

Figure 5 is a top view of an embodiment of a device of the present invention showing the cannula sheath.

Figure 6a is a top view of an embodiment of a device of the present invention showing the cryogenic probe inserted into the cannular cutter and the cannula sheath.

Figure 6b is a top view of an embodiment of a device of the present invention showing the cannular cutter and the cannula sheath retracted proximally to expose the cryogenic probe.

Figure 6c is a top view of an embodiment of a device of the present invention showing the configuration of the device during freezing of tissue with the probe.

Figure 6d is a top view of an embodiment of a device of the present invention showing the cannular cutter and the sheath advancing distally relative to the probe such that the cutter severs a tissue sample after the tissue has been cryogenically treated with the probe.

Figure 6e is a top view of an embodiment of a device of the present invention showing the cutter and sheath retracted proximally so that the severed tissue sample can be removed from the cryogenic probe, such as with tweezers.

Figure 7 is a schematic illustration of an embodiment of a device of the present invention showing the device inserted into breast tissue.

Figure 8 is a schematic illustration of an embodiment of a device of the present invention showing the biopsy device control module and handpiece.

Figure 9 is a schematic illustration of an embodiment of a device of the present invention showing the control module screen.

**Detailed Description of the Invention**

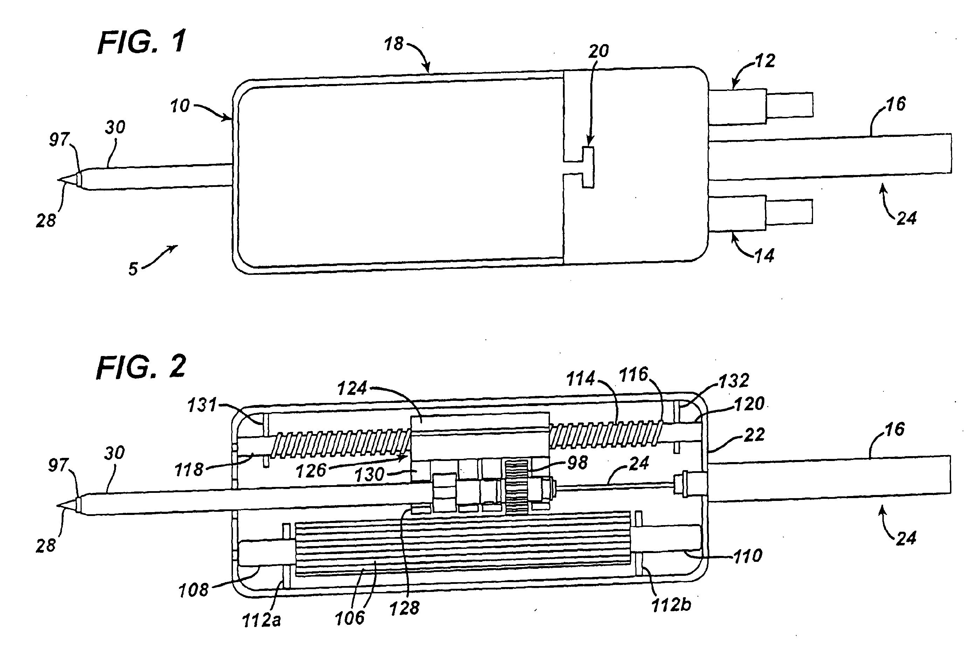

Figures 1 through 5 disclose a biopsy device 5 useful for taking biopsy samples according one embodiment of the present invention. The biopsy device 5 can include a handpiece 10, cryogenic probe 24, cannular cutter 96, and cannular sheath 30.

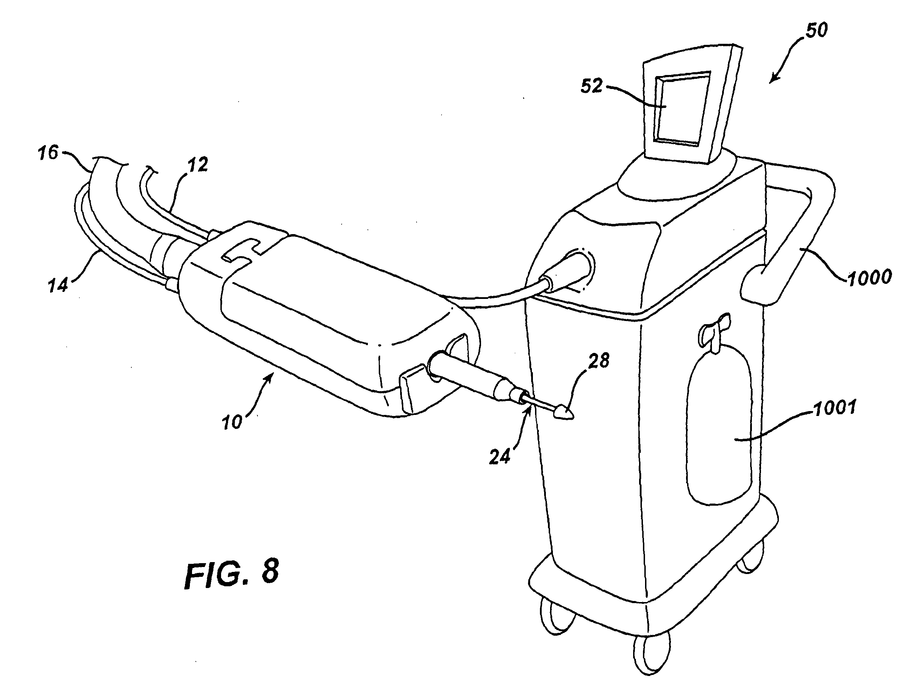

Handpiece 10 can include a lid 18 and latch 20. Handpiece 10 can be configured to receive a longitudinal drive cable 12, a rotational drive cable 14, a cryogen supply tube 16. Longitudinal drive cable 12 can have a distal end which interfaces with handpiece 10, and a proximal end which interfaces with a control module, such as the control module 50 shown in Figure 8.

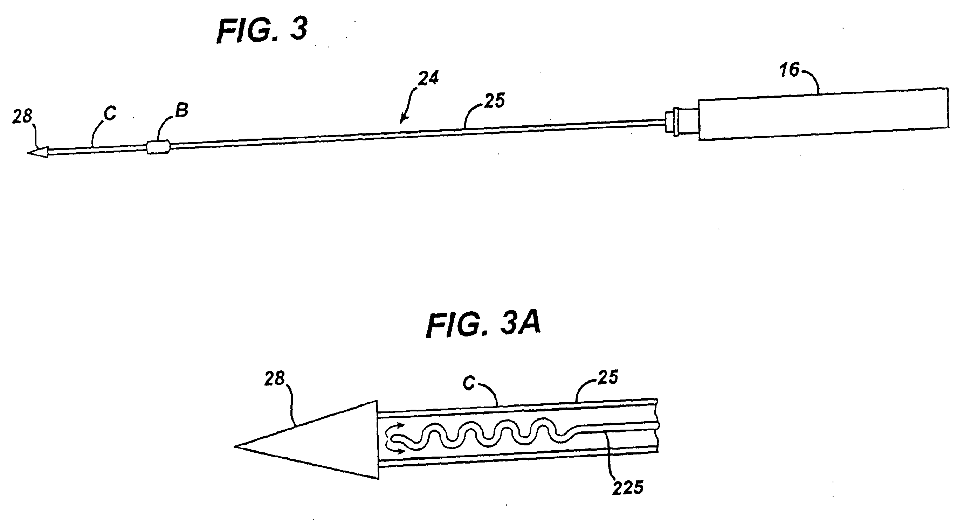

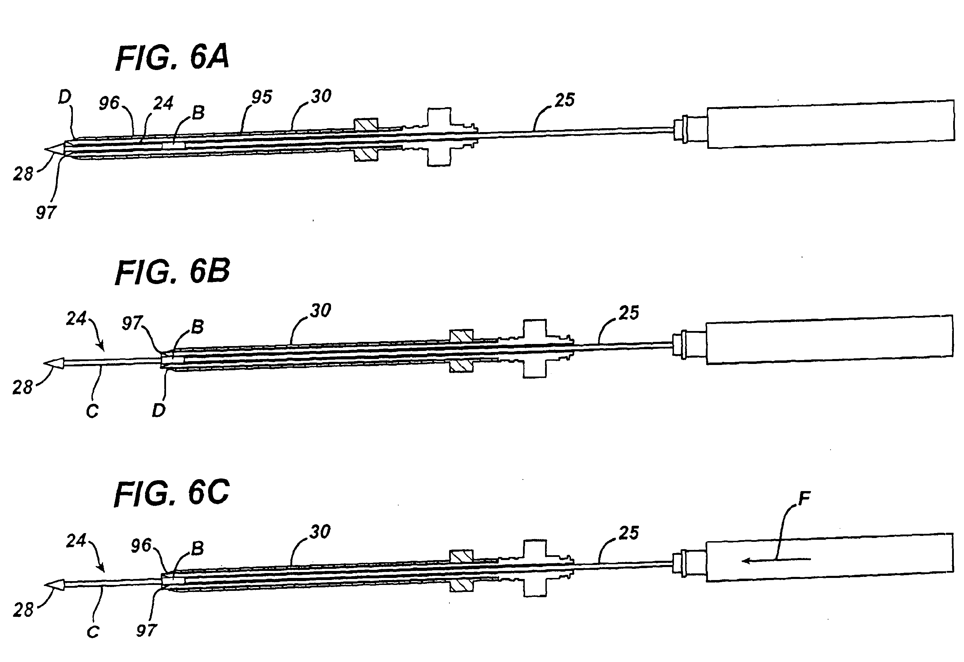

Longitudinal drive cable 12 can be employed to power translational movement of a cannular cutter 96 (Figure 6a). Rotational drive cable 14 can have a distal end which interfaces with handpiece 10, and a proximal end which interfaces with a control module such as control module 50. Rotational drive cable 14 can be employed to power rotational movement of cannular cutter 96. Cryogen supply tube 16 can have a distal end, which interfaces with handpiece 10, and a proximal end, which interfaces with control module 50. Tube 16 supplies a cryogenic substance, such as a cryogenic gas, from control module 50 to cryogenic probe 24 (Figure 3). Lid 18 connects to handpiece 10 by way of latch 20. Lid 18 may be raised to view the internal components of handpiece 10. Handpiece 10 may be constructed so as to be sealed against moisture and contaminants or other debris. If desired, handpiece 10 can be supported by mechanical framework, or other suitable support, to provide for more accurate placement of cryogenic probe 24 within tissue. For instance, if desired, handpiece 10 can be supported for use with a stereotactic table.

Figure 2 illustrates the handpiece 10 with the top portion cut away to reveal the internal components of handpiece 10. Internal components can include a screw 114 having screw features, such as threads 116, and a carriage 124 which as mounted on screw 114 and advancable on screw 114. Carriage 124 can include an internally threaded bore having internal threads engaging the threads 116 of screw 114. A carriage foot 130 can extend from carriage 124. Carriage foot 130 can include a cradle-like recess 128.

A drive gear 104 comprising a first drive axle portion 108 a second drive axle portion 110, and gear teeth 106, is rotatably supported within handpiece 10. Handpiece 10 can include internal axle support rib 112a and internal axle support rib 112b for rotatably supporting the drive axle portions 108 and 110. The drive axle portion 108 extends from a distal end of the drive gear 104 and is supported by axle support rib 112a. Drive axle portion 110 projects from the proximal end of the drive gear 104 and is supported by axle support rib 112b.

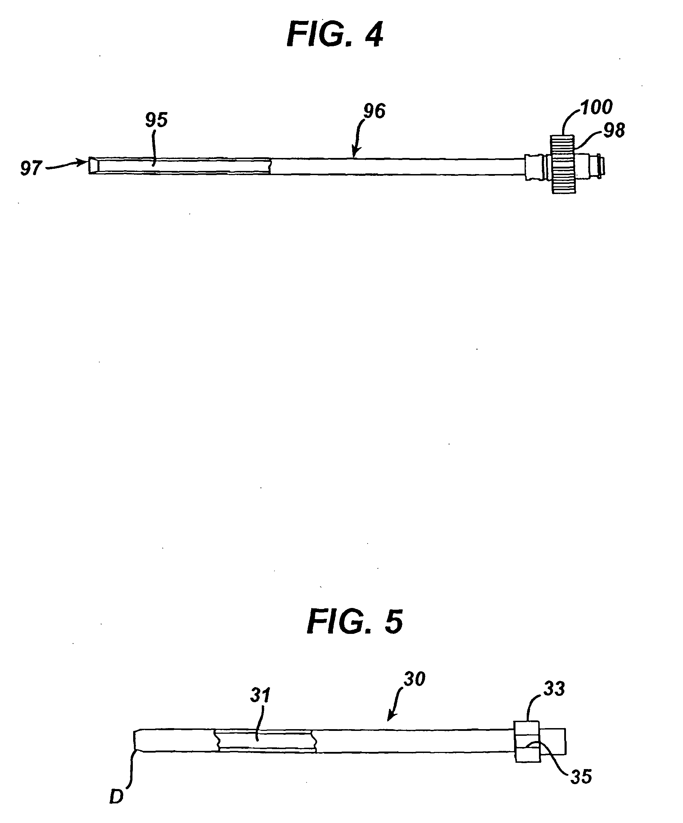

Referring to Figure 4, cannular cutter 96 includes a cutter gear 98 having gear teeth 100 which engage with gear teeth 106 of elongated drive gear 104. Rotation of drive gear 104 rotates cutter gear 98 and the cutter 96, while permitting cutter 96 to translate in a distal or proximal direction. The drive gear 104 can be made of a non metallic material, such as a plastic, including without limitation a liquid crystal polymer. The cannular cutter 96 can also include a distal cutter tip 97.

The carriage 124 supports cannular cutter 96 and cannula sheath 30 such that cannular cutter 96 translates with carriage 124 and can rotate relative to carriage 124, and such that cannula sheath 30 translates with carriage 124 and cannular cutter 96. The cutter tip 97 extends distally beyond the distal end of cannula sheath 30 for tissue cutting exposure.

The carriage 124 is preferably molded from a rigid polymer and can have a generally cylindrically shaped body with a threaded bore extending through the body. Carriage 124 can also include a carriage foot 130 extending from a side of the body. The foot 130 can include a cradle like recess 128 formed into it for rotatably holding the cutter gear 98 in the proper orientation for the cutter gear teeth 100 to mesh properly with the drive gear teeth 106.

The carriage 124 receives elongated screw 114 in threaded bore 126. Elongated screw 114 can be supported in parallel relationship with drive gear 104. Screw 114 can include a distal screw axle 118 projecting from the distal end of screw 114, with axle 118 being rotatably supported by an axle support rib 131 (such as can be formed in the inner surface of the housing of the handpiece 10). The screw 114 can also include a proximal screw axle 120 projecting from the proximal end of screw 114, with axle 120 being supported by axle support rib 132 (such as can be formed in the inner surface of the housing of the handpiece 10).

The screw 114 can include a thread feature 116, such as a lead screw thread, and can be formed of a nonmetallic material, such as a liquid crystal polymer. The rotation of the screw 114 in one direction causes carriage 124 to move distally, while the reverse rotation of the screw 114 causes the carriage 124 to move proximally. In turn, the cutter gear 98, supported by carriage 124, moves distally and proximally according to the direction of the screw rotation, so that the cutter 96 is advanced or retracted relative to the cryogenic probe 24 in a direction generally collinear with the axis of cryogenic probe 24.

In one embodiment, the screw 114 can have a right hand thread feature so that clockwise rotation (looking from the proximal to distal direction) causes the carriage 124 (and cutter 96) to translate in the distal direction. It is also possible to use a left hand thread for the screw 114 as long as provisions are made to do so in the control unit.

Figure 3 illustrates an embodiment of cryogenic probe 24. Cryogenic probe 24 can include a hollow shaft 25, containing within a Joule-Thompson tubing construction, such as the type disclosed in above referenced US Patent 5,522,870. In one embodiment, shown schematically in Figure 3A, an internal tube 225 having an open distal end can be disposed within hollow shaft 25 and extend substantially along the length of hollow shaft 25. The proximal end of the internal tube 225 can be in flow communication with cryogenic supply tube 16. The internal tube 225 provides flow of a cryogenic substance (gas and/or liquid) in a distal direction in hollow shaft 25. The cryogenic substance can exit the distal end of internal tube 225 and flow in proximal direction (as indicated in Figure 3A by arrows) in the space between the inner surface of how shaft 25 and the internal tube 225. The distal most portion of the internal tube 25 associated with a tissue collection area C of the hollow shaft 25 can have a spiral, helical, coiled, serpentine, or other non-straight configuration to increase the surface area of the internal tube 25 adjacent the tissue collection area C. One suitable shaft 25 . A suitable shaft 25 with Joule-Thompson tubing construction can be obtained from Galil Medical, in Woburn, Massachusetts.

The hollow shaft 25 can include a closed, tissue piercing tip 28 at the distal end of the shaft 25. A centering spacer B can be positioned on the shaft 25 and spaced proximally from tip 28. The centering spacer B provides centering of the cutter 96 about shaft 25, such that cutter 96 is supported generally concentrically about shaft 25 as cutter 96 moves moves proximally and distally relative to cryogenic probe 24. The tissue collection area C can be disposed between tip 28 and spacer B. Collection area C can have a diameter smaller than the diameter of spacer B and tip 28, so that the collection area C provides a generally annular space between outside surface of cryogenic probe 24 and the cutter 96 when cutter 96 is advanced in a distal direction over collection area C. Cryogen supply tube 16 can extend from the proximal end of cryogenic probe 24 to a cryogen gas supply, such as a tank 1001 (Figure 8) containing argon.

Figure 4 discloses an embodiment of cutter 96. Cutter 96 can include an elongated hollow tube having a cutter lumen 95 extending along its length. Cutter 96 can also include a sharpened cutter tip 97 at the distal end of the cutter 96. Cutter gear 98 can be disposed at a proximal end of cutter 96. The cutter 96 and gear 98 may be metallic or nonmetallic.
Figure 5 discloses an embodiment of cannula sheath 30. Cannula sheath 30 provides a cover around the cutter 96, such as when cutter 96 is translating and rotating. Cannula sheath 30 can include a sheath lumen 31 extending along the length of the sheath 30, and sheath lumen 31 can be open at the proximal and distal ends of the sheath 30. The inner diameter of sheath lumen 31 is sized to provide a close, sliding fit with the cutter 96, such that cutter 96 can translate and rotate within lumen 31. Sheath 30 can include a tapered distal end D for providing smooth entry into tissue when cryogenic probe 24, cutter 96 and sheath 30 are advanced into tissue. The sheath 30 can be formed of any suitable material including metallic and non-metallic materials. The sheath 30 assists in ensuring that the tissue being severed contacts the rotating cutter 96 only at the edge 97, and helps to shield the tissue from contacting (and potentially wrapping around) the outwardly-facing surface of the cutter 96. A sheath support 33 can be disposed adjacent the proximal end of sheath 30. The sheath support 33 can provide axially facing surfaces which abut adjacent feet 130 of carriage 124. Accordingly proximal and distal motion of the carriage provides proximal and distal motion of the sheath with the cutter 96. The sheath support 33 can include one or more grooves 35 which can engage with a spline, rib, or other protusion (such as can be formed in the housing of handpiece 10) to prevent rotation of sheath 30, while permitting translation of sheath 30 with cutter 96.

Figures 6A-6E illustrate five different steps of the biopsy sequence using the biopsy device of the present invention. Figure 6A illustrates the relative positions of the cryogenic probe 24, cutter 96 and sheath 30 just prior to insertion into tissue. In Figure 6A, the cryogenic probe 24 has been inserted through a proximal opening in the handpiece 10 (handpiece 10 not shown in Figure 6A), with cryogenic probe 24 extending through the cutter lumen 95, and with piercing tip 28 extending just distal of the distal cutting edge 97 of cutter 96. If desired, cryogenic probe 24 can include a threaded connection, or other suitable connection, such as a snap fit or bayonet fitting, for releasably securing the cryogenic probe probe to handpiece 10.

Once cryogenic probe 24 is releasably secured relative to handpiece 10, cryogenic probe 24 does not move relative to handpiece 10. Cutter 96 and sheath 30 can be advanced distally relative to handpiece 10 and cryogenic probe 24 so that edge 97 of the cutter 96 just abuts a proximal face of the piercing tip 28, as shown in Figure 6A. With cryogenic probe 24, cutter 96, and sheath 30 in the position shown in Figure 6A, cryogenic probe 24, cutter 96 and sheath 30 can be advanced into tissue together into tissue.

Figure 6B shows cryogenic probe 24 positioned at a desired site in the tissue mass to be sampled, and with sheath 30 and cutter 96 retracted relative to cryogenic probe 24. Once cryogenic probe 24 has been inserted into the tissue and is located at the desired site within the mass to be biopsied, cannula sheath 30 and cutter 96 are retracted relative to cryogenic probe 24 and handpiece 10 to provide an open configuration of cryogenic probe 24 with tissue collection area C uncovered.

Figure 6C illustrates application of the cryogenic material, such as to provide a "freezing" cycle of the procedure. Figure 6C illustrates flow F of cryogenic material into cryogenic probe 24 to reduce the temperature (e.g freeze) the tissue contacting tissue collection area C. The cryogenic material can be maintained in cryogenic probe 24 for a predetermined period of time, after which the cryogenic material can be evacuated from cryogenic probe 24. The cryogenic material can be employed to cause the tissue to adhere to collection area C of cryogenic probe 24, as well as to provide freezing of the tissue or otherwise provide firming or stiffening of the tissue in preparation cutting of the tissue with cutter 96.

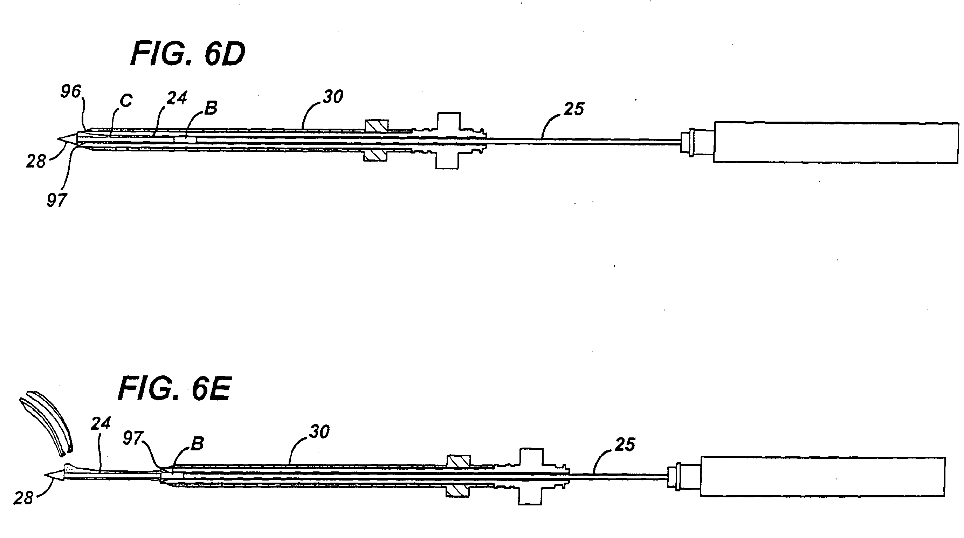

Figure 6D illustrates a cutting phase of the procedure, and shows cryogenic probe 24 with the cutter 96 and sheath advanced over the tissue collection area C of cryogenic probe 24. After the cryogenic gas has been applied and the tissue has adhered to cryogenic probe 24, cutter 96 can be rotatably advanced (for instance, the cutter is advanced distally and simultaneously rotated about its longitudinal axis). The non-rotating cannula sheath 30 can be translated distally simultaneously with cutter 96 to shield the tissue around the biopsy site from contact with the rotating outer surface of the cutter 96. Cutter 96 severs tissue adhered to cryogenic probe 24 (in the annular volume between tissue collection area C and the inner surface of cutter 96) from the surrounding tissue.

Figure 6E illustrates retrieval of the severed tissue sample. Cryogenic probe 24, cutter 96, and sheath 30 are removed from the tissue. Cannula sheath 30 and cutter 96 can then be retracted after cryogenic probe 24 is removed from the tissue mass. Retracting the sheath 30 and cutter 96 exposes the severed tissue sample. If desired, a relative warm material (such as a heated gas or a gas at room temperature) may be applied through cryogenic probe 24 and/or directly to the tissue to facilitate removal of the tissue from the tissue collection area C. In one embodiment, helium can be employed to assist in removing the tissue from cryogenic probe 24. The tissue sample can be removed using tweezers or any other suitable device.

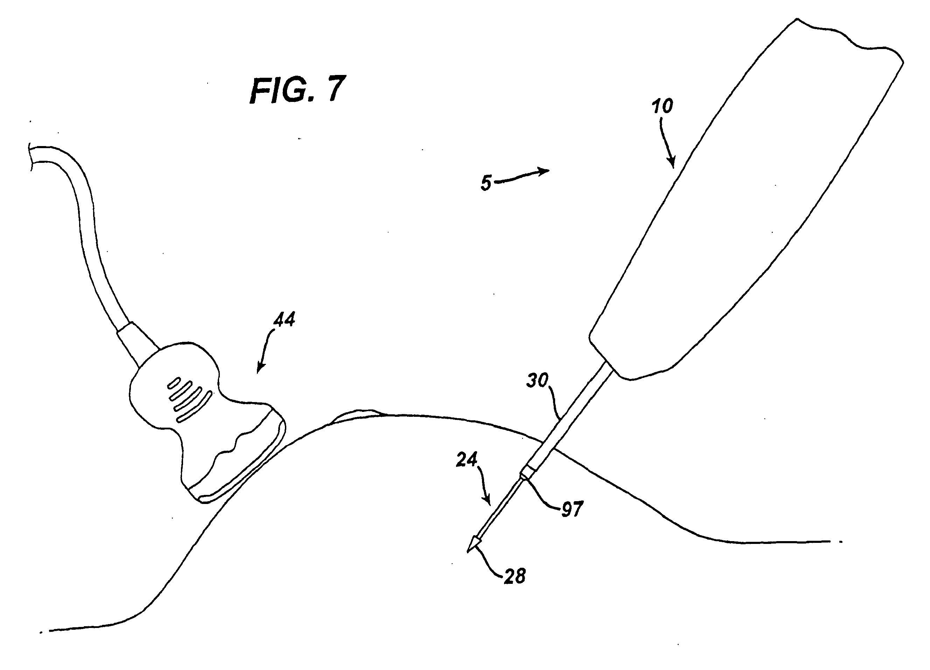

Figure 7 provides a schematic illustration of the biopsy device 5 with cryogenic probe 24 inserted into breast tissue. Sheath 30 and cutter 96 are shown retracted proximally, so that cryogenic probe 24 can cool a tissue sample after the insertion step is completed, with it being understood that sheath 30 and cutter 96 are both advanced distally during the insertion step, and sheath 30 and cutter 96 are retracted proximally once the probe 24 is postioned at the desired tissue site. An imaging device, such as an ultrasound probe 44, may also be used to assist determining proper location of the device with respect to the mass to be biopsied. Suitable imaging devices include, but are not limited to, ultrasound devices, x-ray devices, fluoroscopic devices, and magnetic resonance imaging devices.

Figure 8 illustrates the handpiece 10 with longitudinal drive cable 12, rotational drive cable 14, and supply tube 16 extending from the proximal end of handpiece 10. Longitudinal drive cable 12 and rotational drive cable 14 can be controlled via a control module 50 having a control screen 52. Supply tube 16 can communicate with a supply of cryogenic material, such as a cryogenic gas supply tank 1001. The tank 1001 and the control module 50 can be provided on a movable cart 1000.

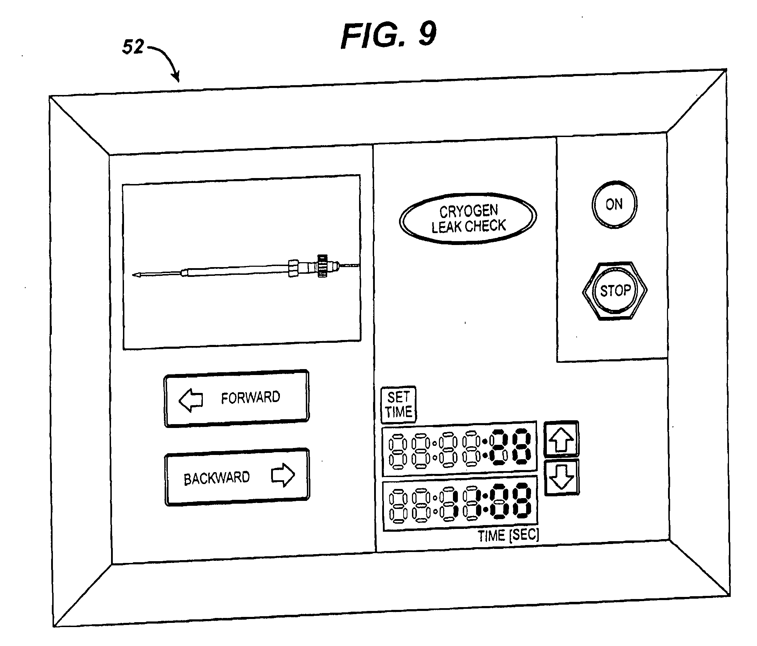

Figure 9 discloses an embodiment of the control screen 52 of control module 50. A Screen 52 may be used to illustrate and/or control the various functions of the device 5 and procedure steps. In Figure 9, the screen 52 includes features illustrating the cutter, sheath, and probe position, as well as features for controlling forward (distal) and backword (proximal) motion of the cutter and sheath. The screen also includes a feature for cryogenic probe set up leak detection, an on/off control, and a time control for cryogenic material application.

The biopsy device of the present invention can be provided for single or multiple use. If desired, one or more of the components of the biopsy device can be disassembled, cleaned, repackaged, and re-sterilized. The various components of the biopsy device can be provided in sterile packaging, either individually, or as a kit.

Various aspects of the present invention have been illustrated, but it will be understood by those skilled in the art that such aspects are only examples and that numerous variations and substitutions are possible without departing from the spirit and scope of the invention. For instance, while the invention has been illustrated with respect to use in breast tissue, the invention may also be used to obtain tissue samples from other tissue types. Additionally, each component of the invention can be alternatively described as a means for providing the function of that component. It is intended that the invention be limited only by the scope and spirit of the appended claims.

## Claims

1. A biopsy device comprising:
a handpiece;
an outer cannular cutter supported for translation relative to the handpiece;
a cryogenic probe disposed at least partially within the outer cannular cutter; and
a cannula sheath disposed around at least a portion of the outer cannular cutter.

2. A biopsy device comprising:
a probe adapted to receive a cryogenic material;
a hollow cutter disposed about at least a portion of the probe, wherein the cutter is movable with respect to the probe to sever tissue; and
a sheath movable with the hollow cutter.

3. The biopsy device of Claim 2 wherein the hollow cutter is translatable and rotatable with respect to the probe.

4. The biopsy device of Claim 2 wherein the sheath is translatable with respect to the probe.

5. The biopsy device of Claim 2 wherein the sheath is non-rotatable with respect to surrounding tissue when the probe, cutter, and sheath are disposed in tissue.

6. The biopsy device of Claim 2 wherein the sheath and hollow cutter are adapted to translate together relative to the probe.

7. A method for providing a biopsy sample comprising the steps of:
positioning a probe into tissue to be sampled;
cooling tissue adjacent to a portion of the probe;
advancing and rotating a hollow cutter over a portion of the probe to sever a sample of the cooled tissue from the surrounding tissue; and
shielding the surrounding tissue from the rotating hollow cutter.

8. The method of Claim 7 wherein the step of cooling comprises freezing the tissue.

9. The method of Claim 7 wherein the step of cooling comprises providing a cryogenic material.

10. The method of Claim 7 further comprising warming the severed tissue sample.

11. The method of Claim 7 wherein the step of shielding the surrounding tissue comprises advancing a non-rotating sheath over a portion of the hollow cutter.

12. The method of claim 11 wherein the step of advancing the non-rotating sheath comprises advancing the non-rotating sheath simultaneously with advancing the hollow cutter.
